# EUROPEAN PATENT SPECIFICATION

(11) **EP 1 545 670 B1**
(45) Date of publication and mention of the grant of the patent: **15.11.2006**
(21) Application number: 03795609.1
(22) Date of filing: 13.08.2003
(51) Int. Cl.: A61M 15/00, B65D 83/14

(54) **AEROSOL DISPENSERS AND ADAPTORS THEREFOR**
AEROSOL-SPENDER UND ADAPTER DAFÜR
DIFFUSEURS D'AEROSOLS ET ADAPTATEURS PREVUS POUR CES DERNIERS

(30) Priority: 16.09.2002 GB 0221343
(43) Date of publication of application: 29.06.2005
(73) Proprietor: 3M Innovative Properties Company, St. Paul, MN 55133-3427 (US)
(72) Inventor: HODSON, Peter, D., Breaston, Derbyshire DE 72 3EL (GB)
(74) Representative: Aleandri-Hachgenei, Lorraine E.
(86) International application number: PCT/US2003/025502
(87) International publication number: WO 2004/024221

(56) References cited:
- WO-A-00/29054
- FR-A- 2 732 883
- US-A- 4 648 393
- US-A- 5 623 920

## Description

### Field of the Invention

This invention relates to adaptors for use in dispensers as well as to dispensers for dispensing doses of pressurized aerosol formulation, in particular for dispensing metered doses of medicament for administration to the respiratory system of a patient.

### Background of the Invention

The use of pharmaceutical aerosols and pressurized medicinal dispensers for inhalation administration of medicaments to a patient is commonplace and becoming increasingly even more important.

The medicament is generally formulated with one or more propellants (e.g. chlorofluorocarbons and more recently hydrogen-containing fluorocarbons, including hydrofluoroalkanes, such as propellant 134a (CF₃CH₂F) and propellant 227 (CF₃CHFCF₃), as well as any additional excipients or components, and then charged in a container or vial. The container is typically fitted by means of a ferrule with a dispensing valve, in particular a metered dose dispensing valve, comprising an elongate outlet member or valve stem movable between closed and dispensing positions, to provide a dispensing canister. The dispensing canister is typically used in conjunction with an adaptor, typically having a patient port, for example a mouthpiece or a port adapted for nasal use. In conventional dispensers, such as a conventional press-and-breathe inhaler, the adaptor typically comprises a support block having a socket adapted to receive the valve stem of the dispensing valve and an orifice having open communication with the socket and the patient port.

With conventional dispensing valves, the valve stem is typically biased to its closed position with the valve stem extending outwardly relative to the container (an "outer" closed position) and to actuate or fire the valve, the valve stem is depressed inwardly to the dispensing position (an "inner" dispensing position) allowing a dose to be dispensed. For example, with a conventional press-and-breathe device, the patient fires the device by depressing the container towards the support block of the adaptor and this depressing the valve stem inwardly, while inhaling.

Some dispensing valves, for example certain embodiments of shuttle-type metered dose dispensing valves disclosed in US 5,772,085, do not operate in the conventional push-to-fire manner. Instead the valve stem moves relative to the container from an inner closed or priming position to an outer dispensing position. In other words the dispensing valve is actuated or fired upon movement of the valve stem outwardly relative to the container. The operation of such valves would typically require the user to depress the valve stem, inwardly to the priming position and then release to allow firing during the release stroke upon the outward movement of the valve stem to the dispensing position.

WO 00/23054 discloses breath-actuated aerosol dispensers for use with both conventional and release-to-fire valves.

### Summary of the Invention

It has been recognized that the unfamiliar firing upon release (release to-fire operation) may cause confusion for the patient. In particular, it has been appreciated that the patient often has undergone intensive training to learn how to coordinate inhaling while operating a conventional press-and-breathe type of inhaler (i.e. a push-to-fire dispenser), and thus may find it difficult, if not nearly impossible, to switch to using a dispenser based on a release-to-fire operation. This holds particularly true, because patients, such as asthma patients, often tend to be sensitive to and reluctant towards any change in the medicinal dispenser (or inhaler) they are using.

Surprisingly we have been able to provide adaptors as well as dispensers, which allow the patient to use release-to-fire-type valves in the conventional manner.

Accordingly in one aspect of the present invention there is a provided an adaptor for use with a container equipped with a dispensing valve that comprises a valve stem movable between an inner closed or priming position and an outer dispensing position, for dispensing doses of pressurized aerosol formulation,
the adaptor being adapted to receive the container and comprising an actuation mechanism, said actuation mechanism being arranged such that the user will operate the mechanism by applying a depressive or squeezing force and the dose will be dispensed upon said depressive or squeezing force and said actuation mechanism being arranged such that upon release of the mechanism by the user, the valve stem of the dispensing valve will be moved automatically to its closed or priming position.

In another aspect of the present invention there is provided a dispenser for dispensing doses of pressurized aerosol formulation, said dispenser comprising:
a container containing pressurized aerosol formulation and equipped with a dispensing valve that comprises a valve stem movable between an inner closed or priming position and an outer dispensing position, to dispense a dose; and
an adaptor adapted to receive the container and comprising an actuation mechanism, said actuation mechanism being arranged such that the user operates the mechanism by applying a depressive or squeezing force and the dose is dispensed upon said depressive or squeezing force and said actuation mechanism being arranged such that upon release of the mechanism by the user, the valve stem of the dispensing valve is moved automatically to its closed or priming position.

The use of adaptors or dispensers in accordance with the invention is advantageous in that the patient can operate the dispenser in a similar manner to a conventional press-and-breathe type inhaler. While inhaling, the patient exerts a depressing force upon the actuation mechanism to allow a dose to dispense. After such firing, the patient simply releases the actuation mechanism. Because the actuation mechanism is arranged such that the valve stem of the dispensing valve is moved or returned automatically to its inner closed or priming position upon said release, the dispenser is advantageously ready for the next firing without any additional operation by the patient. Dispensers in accordance with the invention are also advantageous in that due to the automatic immediate resetting of the valve to its inner closed or priming position upon release by the patient, potentially sensitive portions of the interior of the dispensing valve are desirably sealed off from the outside environment, and thus protected from air and moisture ingress between individual firings of the dispenser.

In preferred embodiments, the actuation mechanism is arranged, such that upon said depressive or squeezing force the valve stem is pulled from its closed or priming position into its dispensing position. Such preferred embodiments are particularly advantageous for use with dispensing valves free of an internal spring bias for biasing the valve stem towards its outer dispensing position.

Suitable dispensing valves include in particular dispensing valves arranged to be of neutral bias (i.e. the valve stem being neither biased towards its closed/priming or dispensing position) as well as dispensing valves arranged, such that the valve stem will be biased outwardly towards its dispensing position by vapor pressure generated by pressurized aerosol formulation contained within the container of the dispenser.

In one embodiment, the actuation mechanism comprises a mounting block and an actuating member; said mounting block being adapted to retain the container and to prevent movement of the container relative to the adaptor and said actuating member being in contact with the valve stem and biased towards the container, such that the valve stem rests in its closed or priming position. Suitably the actuating member may be arranged, such that upon said depressive or squeezing force the actuating member moves away from the container against the bias allowing the valve stem to move into its dispensing position. Alternatively and more desirably, the actuating member may be coupled to the valve stem and arranged, such that upon said depressive or squeezing force the actuating member moves away from the container against the bias, thereby pulling the valve stem into its dispensing position.

In other embodiments, the actuation mechanism may comprise an anchoring block, which is adapted to retain the valve stem and to prevent movement of the valve stem relative to the adaptor. Here the container is desirably biased towards the valve stem, such that the valve stem rests in its closed or priming position, and the actuation mechanism desirably comprises an actuating member being arranged, such that upon said depressive or squeezing force, the actuating member moves the container against the bias away from the valve stem, thereby pulling the valve stem into its dispensing position relative to the container.

These adaptors and dispensers are particularly suitable for use with metered dose dispensing valves, more particularly shuttle-type metered dose dispensing valves. In preferred embodiments, the dispensing valve further comprises a chamber and an outlet passage, wherein the valve stem extends into the chamber and is movable relative to the chamber between the closed or priming position and the dispensing position; the valve stem having a configuration including an external surface and the chamber having an internal configuration including an internal surface, such that a movable metered volume of pressurized aerosol formulation is capable of being defined therebetween and such that during movement between the closed or priming position and the dispensing position the valve stem sequentially:
(i) allows free flow of aerosol formulation into and out of the chamber,
(ii) defines a closed metered volume for pressurized aerosol formulation between the external surface of the valve stem and internal surface of the chamber, and
(iii) moves with the closed metered volume within the chamber without decreasing the volume of the closed metered volume until the metered volume communicates with the outlet passage thereby allowing dispensing of the metered volume of pressurized aerosol formulation.

Further embodiments of the invention are defined in the dependent claims.
The invention, its embodiments and further advantages will be described in the following with reference to the following drawings.

### Brief Description of the Drawings

Figures 1a and b represent vertical cross sections through a valve comprising an valve stem movable from an inner closed or priming position to an outer dispensing position.
Figure 2 represents schematically a vertical cross section of a preferred embodiment of the dispenser in accordance with the invention.
Figures 3 represents schematically a vertical cross section of another preferred embodiment of the dispenser in accordance with the invention.
Figure 4 represents schematically a vertical cross section of an additional preferred embodiment of the dispenser in accordance with the invention.
Figure 5 represents schematically a vertical cross section of a further preferred embodiment of the dispenser in accordance with the invention.
Figure 6 represents schematically a vertical cross section of a yet further preferred embodiment of the dispenser in accordance with the invention.

### Detailed Description

It is to be understood that the present invention covers all combinations of particular and preferred aspects of the invention described herein.

For a better understanding of the various aspects of the present invention, one exemplary dispensing valve, which is actuated or fired upon movement of the valve stem outwardly and particularly suitable for use in the present invention, will be initially described.

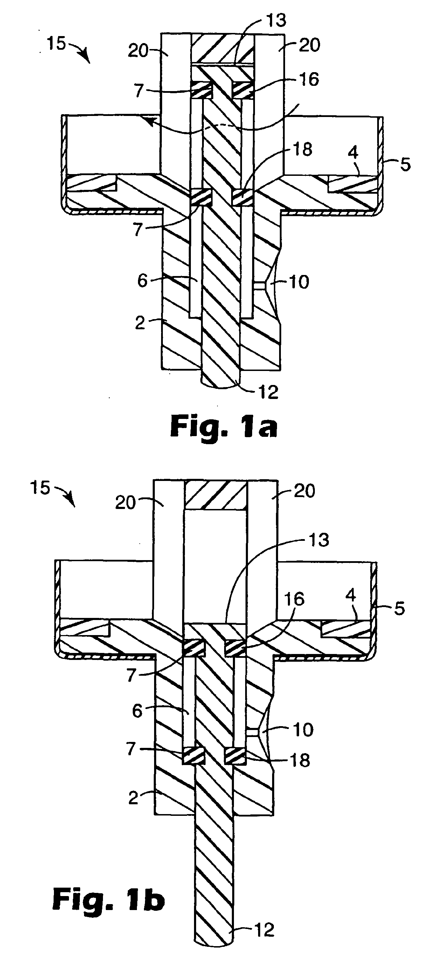

Figures 1a and b illustrate an exemplary shuttle-type metered dose dispensing valve. Referring to Fig. 1a and b, the valve typically comprises a body (2) having an annular seal or gasket (4) for engaging the neck of an aerosol container or vial (not shown) to facilitate a gas-tight seal. The body (2) may be secured to the aerosol container or vial by any suitable means e.g. a conventional outer casing or ferrule (5), which is crimped around the neck of the aerosol container. As can be best seen in Fig. 1b, the body (2) defines a chamber (6) having an outlet passage (10) for dispensing e.g. pressurized aerosol formulation. The valve stem (12) extends through the chamber (6) and is movable between a closed or priming position (as shown in Fig. 1a) and a dispensing position (as shown in Fig. 1b). The valve stem (12) is desirably fitted with an inner seal (16) and outer seal (18), which provide gas-tight seals between the valve stem and the inner wall of the chamber (6). The chamber (6), external dimensions of the valve stem (12) and the positions of the seals (16 and 18) are arranged to define a pre-determined volume within the chamber (6) between the seals (16 and 18). This can be best understood by reference to Fig. 1b showing the valve in its dispensing position. As can be seen in Fig. 1a, in its closed or priming position the space between the seals (16 and 18) around the valve stem (12) extends into the reservoir containing aerosol formulation. As the valve stem (12) moves downwardly to its dispensing position, the seal (18) moves down the chamber allowing free access of the aerosol formulation in to the chamber (6). Further movement of the valve stem causes seal (16) to enter the chamber (6) thereby trapping a metered volume of aerosol formulation between the seals (16 and 18) and the interior wall of the chamber (6). When the valve stem reaches its dispensing position the seal (18) passes outlet passage (10) thereby allowing direct communication between the metered volume and the outlet passage (10) thereby allowing the metered volume of formulation to be dispensed. In the illustrated valve, the valve is arranged, in particular the cross-sectional area of the seals (16 and 18) is arranged, such that the valve stem will be biased outwardly towards its dispensing position by vapor pressure generated by pressurized aerosol formulation contained within the container of the dispenser. The alignment of the valve stem may be ensured by ribs (20) which do not obstruct the free flow of aerosol formulation (as depicted by the arrow in Figure 1a) around the valve stem (12) between the seals (16 and 18).

It is to be understood that Figures la and b show one exemplary dispensing valve, which is suitable for use in the present invention, and that other dispensing valves, in particular other metered dose dispensing valves, having a valve stem movable between an inner closed or priming position and an outer dispensing position may also be suitable for use. Further examples of dispensing valves, which are actuated or fired upon movement of the elongate outlet member or valve stem outwardly, are described in US 5,772,085, the contents of which are incorporated herein by reference. Other examples of dispensing valves, which are actuated or fired upon movement of an elongate outlet member or valve stem are described in US 2,980,301; US 3,176,887; US 3,176,889;; US 3,591,059; and US 4,506,803.

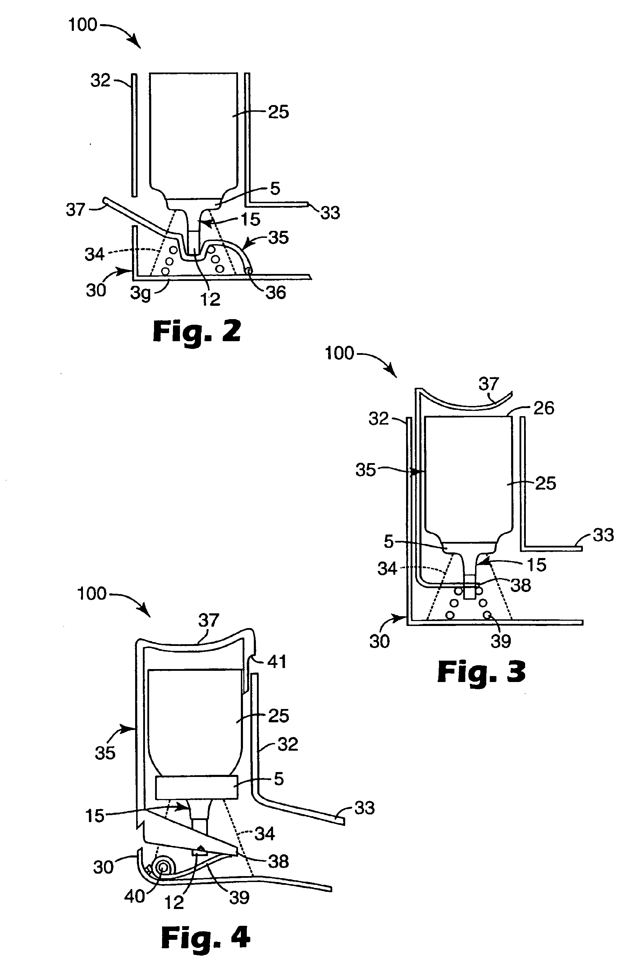

Figure 2 shows schematically a vertical cross-section of a preferred embodiment of a dispenser (100) in the form of an inhaler incorporating a preferred embodiment of an adaptor (30). The adaptor comprises an elongate or cylindrical portion (32), adapted to received the aerosol container (25) equipped with a dispensing valve (generally indicated at 15) comprising a valve stem (12) movable between an inner closed or priming position and an outer dispensing position. The dispensing valve is typically fitted onto the container by means of a ferrule (5) with the valve stem extending beyond the ferrule. The adaptor also typically comprises a mouthpiece portion (33). A mounting block (34) is provided that supports and retains the container (25), in particular at the position of the ferrule (5), preventing movement of the container. The mounting block (34) is desirably arranged, such that the outlet passage (not shown) of the dispensing valve is aligned along the axis of the mouthpiece portion (33). The adaptor includes an actuation member (35) provided in the form of a lever, which is mounted on a pivot (36) and has a user end (37), which extends externally from the adaptor, in particular from the elongate or cylindrical portion (32) of the adaptor. The actuating member (35) is biased upwardly towards the container (25) by the action of a conical compression spring (39) mounted between the actuating member and an internal surface of the adaptor. The outer end of the valve stem (12) rests against the actuating member (35), and because the member is biased upwardly towards the container, the valve stem normally rests in its closed or priming position.

In operation, the patient presses downwards on the user end (37) of the actuating member (35), whilst simultaneously inhaling through the mouthpiece portion (33). In so doing, the patient causes the actuating member (35) to pivot downwards against the action of the spring (39), thereby allowing the valve stem (12) to move outwardly into its dispensing position.

Desirably the dispensing valve is arranged, such that the valve stem is biased outwardly towards its dispensing position by vapor pressure generated by pressurized aerosol formulation contained within the container of the dispenser. Thus, the valve stem moves outwardly into its dispensing position under the influence of the internal vapor pressure. Alternatively, the dispensing valve may include an internal spring bias positioned at the inner end of the valve stem (not shown) biasing the valve stem towards its dispensing position. In this case, the valve stem moves outwardly into its dispensing position under the influence of the internal spring bias.

After taking a dose of medicament, the patient simply releases the actuating member (35), allowing the spring (39) to return the actuating member to its rest position and thus the valve stem (12) into its closed or priming position. This reset action occurs automatically as soon as the patient releases the actuating member (35), thereby minimizing the time in which internal portions of the dispensing valve, such as a virtual metering chamber, may be open to air or atmospheric moisture. It will be appreciated that in embodiments including a dispensing valve comprising an internal spring bias, the spring of the adaptor will be selected to exert, over the range of spring extensions corresponding to the return of the valve stem from its outer dispensing position to its closed or priming position, a suitably greater compressive force that that exerted by the internal spring.

Figure 3 shows schematically a vertical cross-section of another preferred embodiment of a dispenser (100) in the form of an inhaler incorporating another preferred embodiment of an adaptor (30). Similar to the dispenser illustrated in Figure 2, the adaptor comprises an elongate or cylindrical portion (32), adapted to received the aerosol container (25) equipped with a dispensing valve (generally indicated at 15) comprising a valve stem (12) movable between an inner closed or priming position and an outer dispensing position. The adaptor also includes a mouthpiece portion (33) as well as a mounting block (34) that supports and retains the container, in particular at the position of the ferrule (5), preventing movement of the container. The adaptor includes an actuating member (35) in the form of a slider. The actuating member (35), which in its cross-section is essentially C-shaped, is located within a gap between the elongate or cylindrical portion (32) and the container (25), with a user end (37) extending outwardly from the open end of the elongate or cylindrical portion (32) and the other end, the internal end, (38) gripping the valve stem. The user end (37) of the actuating member is preferably provided in the form of a concave recess extending over the closed end (26) of the container. A conical compression spring (39) is mounted between the internal end of the actuating member (35) and an internal surface of the adaptor, such that the actuating member is biased upwardly towards the container, so that the valve stem (12) normally rests in its closed or priming position.

In operation, the patient depresses the user end (37) of the actuating member (35), typically by placing their finger(s) in the concave recess of the user end, whilst simultaneously inhaling through the mouthpiece portion (33). In so doing, the patient causes the actuating member (35) to move downwards against the action of the spring (39), thereby pulling the valve stem (12) into its dispensing position.

The arrangement of the actuation mechanism, such that upon a depressive or squeezing force applied by the user the valve stem is pulled into its dispensing position (referred to as "positive pull out action" in the following) is advantageous in that it guarantees the provision of enough force to move the valve stem (12) without the need for an internal spring bias within the dispensing valve. Thus, potential problems associated with the immersion of a spring in a medicinal aerosol formulation can be avoided. Such potential problems may include for example medicament deposition on the large and flexing surface area of the spring, chemical interactions between the drug and the spring, and hindrance to the free flow of aerosol formulation into and out of the chamber. This embodiment and other embodiments (e.g. as described below) including positive pull-out action are particularly desirable for use with dispensing valves having neutral bias or dispensing valves in which the valve stem is biased towards its dispensing position only by vapor pressure generated by the pressurized aerosol formulation contained in the container.

After taking a dose of medicament, the patient simply releases the actuating member (35), allowing the spring (39) to automatically return the actuating member to its rest position and thus the valve stem (12) to its inner closed or priming position.

Figure 4 shows schematically a vertical cross-section of a variation of the preferred embodiment of the dispenser (100) shown in Figure 3. This dispenser is generally similar to the dispenser illustrated in Figure 3, except that the spring (39) is in the form of a torsion spring mounted via a post (40) on the adaptor (30). The user end (37) of the actuating member (35) additionally comprises a movement stop (41), which will act against the top rim of the elongate or cylindrical portion (32) of the adaptor (30). This stop (41) desirably limits and defines the maximum stem movement possible. The other end of the actuating member (35), the internal end (38), is fixedly located on the valve stem (12). The principles of operation are analogous with those described in conjunction with the dispenser shown in Figure 3.

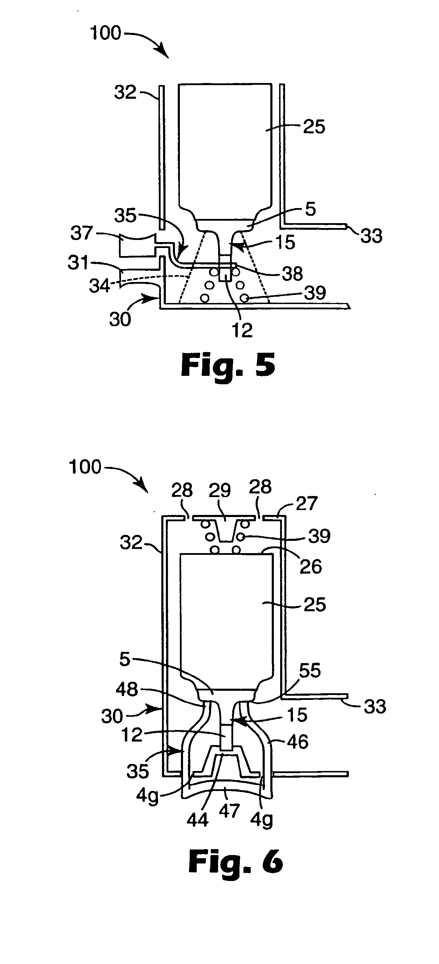

Figure 5 shows schematically a vertical cross-section of an additional preferred embodiment of a dispenser (100) in the form of an inhaler incorporating an additional preferred embodiment of an adaptor (30). This dispenser is similar to the dispenser illustrated in Figure 3, with an exception that the actuating member (35) has a different shape. In particular, the user end (37) of the actuating member, which is desirably in the form of a finger-button, extends outwardly from a side wall of the of the adaptor, in particular the elongate or cylindrical portion (32) thereof. In addition, an extension (31), also desirably in the form of a finger-bottom, is provided on an external surface of the adaptor in a corresponding position to allow its use in coordination with the user end (37) of the actuating member (35). The patient operates the actuation mechanism by squeezing together the user end (37) of the actuating member (35) and the extension (31). The principles of operation are analogous with those described in conjunction with the dispenser shown in Figure 3.

Figure 6 shows schematically a vertical cross-section of a further preferred embodiment of a dispenser (100) in the form of an inhaler incorporating a further preferred embodiment of an adaptor (30). Similar to the other embodiments illustrated in Figures 2 to 5, the adaptor (30) comprises an elongate or cylindrical portion (32), adapted to receive the aerosol container (25) equipped typically by means of a ferrule (5) with a dispensing valve (generally indicated at 15) comprising a valve stem (12) movable between an inner closed or priming position and an outer dispensing position. In this embodiment, the adaptor is provided with an anchoring block (44), which retains the outer end of the valve stem (12) and prevents movement of the valve stem relative to the adaptor. The anchoring block (44) is desirably arranged, such that the outlet orifice (not shown) of the dispensing valve is aligned along the axis of the mouthpiece portion (33). The elongate or cylindrical portion (32) of adaptor includes an end-cap (27) over the closed end (26) of the container (25). For airflow, orifices (28) may be provided in the end-cap as shown or alternatively in the adaptor near the anchoring block. A conical compression spring (39) is mounted on a guide post (29) between an internal surface of the end-cap (27) and an outer surface of the closed end (26) of the container, such that the container is biased downwardly towards the valve stem, so that the valve stem (12) normally rests in its inner closed or priming position. The adaptor includes an actuating member (35), which is essentially U-shaped in its cross-section. The actuating member is mounted, such that the base portion (47) of the member is located adjacent to an external surface of the adaptor and the side wall or side fingers (46) extend(s) into the interior of the adaptor. The upper edge (48) of the side wall or side fingers is adjacent to the underside of the container (25), in particular the underside of the shoulder (55) of the ferrule (5). The base portion (47), which serves as a user portion of the actuating member, is desirably in the form of a concave recess or thumb-button. For ease in inserting the side wall or side fingers (46) through appropriate slots (49) in the adaptor body, the side wall or fingers (46) may be flexible. The side wall or side fingers (46) may be provided with projections (not shown) to prevent the removal or detachment of the actuating member (35) from the adaptor body by the patient.

In operation, the patient presses the base portion (47) of the actuating member (35) towards the adaptor body, whilst simultaneously inhaling through the mouthpiece portion (33). In so doing, the patient causes the actuating member (35) to push the container (25) upwardly against the action of the spring (39), thereby pulling (in a relative movement) the valve stem (12) into its dispensing position. After taking a dose of medicament, the patient simply releases the base portion (47) of the actuating member (35), allowing the spring (39) to automatically return the container (25) to its rest position and thus the valve stem (12) in its inner closed or priming position.

It will be understood that the present disclosure of particular preferred embodiments in accordance with the invention is for the purpose of illustration only and the invention extends to modifications, variations and improvements thereof.

## Claims

1. An adaptor (30) for use with a container (25) equipped with a dispensing valve (15) that comprises a valve stem (12) movable between an inner closed or priming position and an outer dispensing position, for dispensing doses of pressurized aerosol formulation,
the adaptor being adapted to receive the container and comprising an actuation mechanism, **characterized in that** said actuation mechanism is arranged such that the user will operate the mechanism by applying a depressive or squeezing force and the dose will be dispensed upon said depressive or squeezing force and wherein said actuation mechanism is arranged such that upon release of the mechanism by the user, the valve stem of the dispensing valve will be moved automatically into its closed or priming position.

2. An adaptor according to claim 1, wherein the actuation mechanism is arranged, such that upon said depressive or squeezing force the valve stem (12) will be pulled from its closed or priming position into its dispensing position.

3. An adaptor according to claim 1 or claim 2, wherein the actuation mechanism comprises a mounting block (34) and an actuating member (35); said mounting block being adapted to retain the container (25) and to prevent movement of the container relative to the adaptor (30) and said actuating member being arranged to be in contact with the valve stem (12) and biased towards the container, such that the valve stem will rest in its closed or priming.

4. A dispenser (100) for dispensing doses of pressurized aerosol formulation to a user, said dispenser comprising:
a container (25) containing a pressurized aerosol formulation and equipped with a dispensing valve (15) that comprises a valve stem (12) movable between an inner closed or priming position and an outer dispensing position, to dispense a dose; and
an adaptor (30) adapted to receive the container and comprising an actuation mechanism, **characterized in that** said actuation mechanism is arranged such that the user operates the mechanism by applying a depressive or squeezing force and the dose is dispensed upon said depressive or squeezing force and wherein said actuation mechanism is arranged such that upon release of the mechanism by the user, the valve stem of the dispensing valve is moved automatically to its closed or priming position.

5. A dispenser according to claim 4, wherein the actuation mechanism is arranged, such that upon said depressive or squeezing force the valve stem is pulled from its closed or priming position into its dispensing position.

6. A dispenser according to claim 4 or 5, wherein the actuation mechanism comprises a mounting block (34) and an actuating member (35); said mounting block being adapted to retain the container (25) and to prevent movement of the container relative to the adaptor (30) and said actuating member being in contact with the valve stem (12) and biased towards the container, such that the valve stem rests in its closed or priming.

7. A dispenser according to claim 6 as dependent on claim 4, wherein said actuating member (35) is arranged, such that upon said depressive or squeezing force the actuating member moves away from the container (25) against the bias allowing the valve stem (12) to move into its dispensing position.

8. A dispenser according to claim 6 as dependent on claim 5, wherein said actuating member (35) is coupled to the valve stem (12) and wherein said actuating member is arranged, such that upon said depressive or squeezing force the actuating member moves away from the container (25) against the bias and the valve stem is pulled into its dispensing position.

9. A dispenser according to claim 5, wherein the actuation mechanism comprises an anchoring block (44), which is adapted to retain the valve stem (12) and to prevent movement of the valve stem relative to the adaptor (30), and wherein the container (25) is biased towards the valve stem, such that the valve stem rests in its closed or priming position, and wherein said actuation mechanism further comprises an actuating member (35), said actuating member being arranged, such that upon said depressive or squeezing force, the actuating member moves the container against the bias away from the valve stem, thereby pulling the valve stem into its dispensing position relative to the container.

10. A dispenser according to any one of claims 4 to 9, wherein the dispensing valve (15) is arranged, such that the valve stem (12) is biased towards its dispensing position by vapor pressure generated by the pressurized aerosol formulation.

11. A dispenser according to any one of claims 4, 5, 6, 8 and 9, wherein the dispensing valve (15) is of neutral bias.

12. A dispenser according to claim 7, wherein the dispensing valve (15) comprises an internal spring bias (39) positioned at the inner end of the valve stem biasing the valve stem towards its dispensing position.

13. A dispenser according to any one of claims 4 to 12, wherein the dispensing valve (15) is a metered dose dispensing valve.

14. A dispenser according to claim 13, wherein the dispensing valve (15) further comprises a chamber (6) and an outlet passage (10), and
the valve stem (12) extends into the chamber and is movable relative to the chamber between the closed or priming position and the dispensing position, the valve stem having a configuration including an external surface and the chamber having an internal configuration including an internal surface, such that a movable metered volume of pressurized aerosol formulation is capable of being defined there between and such that during movement between the closed or priming position and the dispensing position the valve stem sequentially:
(i) allows free flow of aerosol formulation into and out of the chamber,
(ii) defines a closed metered volume for pressurized aerosol formulation between the external surface of the valve stem and internal surface of the chamber, and
(iii) moves with the closed metered volume within the chamber without decreasing the volume of the closed metered volume until the metered volume communicates with the outlet passage thereby allowing dispensing of the metered volume of pressurized aerosol formulation.

15. A dispenser according to any one of claims 4 to 14, wherein the pressurized aerosol formulation comprises medicament

16. A dispenser according to any one of claims 4 to 15, wherein the pressurized aerosol formulation comprises a propellant.

17. A dispenser according to claim 16, wherein the propellant is a hydrofluoroalkane.

18. A dispenser according to claim 17, wherein the propellant is selected from HFA 134a, HFA 227 and mixtures thereof.

19. A dispenser according to any one of claims 4 to 18, wherein the dispenser is in the form of an inhaler.

## Patentansprüche

1. Adapter (30) zur Verwendung mit einem Behälter (25), der mit einem Abgabeventil (15) ausgestattet ist, welches eine Ventilspindel (12) aufweist, die zwischen einer inneren geschlossenen oder Grundposition und einer äußeren Spendeposition beweglich ist, zum Spenden von Dosen von unter Druck stehender Aerosol-Formulierung,
wobei der Adapter so beschaffen ist, dass er den Behälter aufnehmen kann, und einen Betätigungsmechanismus aufweist, **dadurch gekennzeichnet, dass** der Betätigungsmechanismus so gestaltet ist, dass der Benutzer den Mechanismus betätigt, indem er eine Druck- oder Quetschkraft anwendet, und die Dosis bei Einwirkung der Druck- oder Quetschkraft gespendet wird, und wobei der Betätigungsmechanismus so gestaltet ist, dass beim Loslassen des Mechanismus durch den Benutzer die Ventilspindel des Abgabeventils automatisch in ihre geschlossene oder Grundposition bewegt wird.

2. Adapter nach Anspruch 1, wobei der Betätigungsmechanismus so gestaltet ist, dass bei Einwirkung der Druck- oder Quetschkraft die Ventilspindel (12) aus ihrer geschlossenen oder Grundposition in ihre Spendeposition gezogen wird.

3. Adapter nach Anspruch 1 oder Anspruch 2, wobei der Betätigungsmechanismus einen Halterungsblock (34) und ein Betätigungselement (35) aufweist; wobei der Halterungsblock so beschaffen ist, dass er den Behälter (25) hält und eine Bewegung des Behälters relativ zu dem Adapter (30) verhindert, und wobei das Betätigungselement so gestaltet ist, dass es sich mit der Ventilspindel (12) in Kontakt befindet und zum Behälter hin vorgespannt ist, derart, dass die Ventilspindel in ihrer geschlossenen oder Grundposition verbleibt.

4. Spender (100) zum Spenden von Dosen von unter Druck stehender Aerosol-Formulierung, wobei der Spender aufweist:
einen Behälter (25), der eine unter Druck stehende Aerosol-Formulierung enthält und mit einem Abgabeventil (15) ausgestattet ist, welches eine Ventilspindel (12) aufweist, die zwischen einer inneren geschlossenen oder Grundposition und einer äußeren Spendeposition beweglich ist, um eine Dosis zu spenden; und
einen Adapter (30), der so beschaffen ist, dass er den Behälter aufnehmen kann, und einen Betätigungsmechanismus aufweist, **dadurch gekennzeichnet, dass** der Betätigungsmechanismus so gestaltet ist, dass der Benutzer den Mechanismus betätigt, indem er eine Druck- oder Quetschkraft anwendet, und die Dosis bei Einwirkung der Druck- oder Quetschkraft gespendet wird, und wobei der Betätigungsmechanismus so gestaltet ist, dass beim Loslassen des Mechanismus durch den Benutzer die Ventilspindel des Abgabeventils automatisch in ihre geschlossene oder Grundposition bewegt wird.

5. Spender nach Anspruch 4, wobei der Betätigungsmechanismus so gestaltet ist, dass bei Einwirkung der Druck- oder Quetschkraft die Ventilspindel aus ihrer geschlossenen oder Grundposition in ihre Spendeposition gezogen wird.

6. Spender nach Anspruch 4 oder 5, wobei der Betätigungsmechanismus einen Halterungsblock (34) und ein Betätigungselement (35) aufweist; wobei der Halterungsblock so beschaffen ist, dass er den Behälter (25) hält und eine Bewegung des Behälters relativ zu dem Adapter (30) verhindert, und wobei das Betätigungselement sich in Kontakt mit der Ventilspindel (12) befindet und zum Behälter hin vorgespannt ist, derart, dass die Ventilspindel in ihrer geschlossenen oder Grundposition verbleibt.

7. Spender nach Anspruch 6, als von Anspruch 4 abhängig, wobei das Betätigungselement (35) so gestaltet ist, dass bei Einwirkung der Druck- oder Quetschkraft das Betätigungselement sich entgegen der Vorspannung von dem Behälter (25) weg bewegt und dabei der Ventilspindel (12) ermöglicht, sich in ihre Spendeposition zu bewegen.

8. Spender nach Anspruch 6, als von Anspruch 5 abhängig, wobei das Betätigungselement (35) mit der Ventilspindel (12) gekoppelt ist und wobei das Betätigungselement so gestaltet ist, dass bei Einwirkung der Druck- oder Quetschkraft das Betätigungselement sich entgegen der Vorspannung von dem Behälter (25) weg bewegt und die Ventilspindel in ihre Spendeposition gezogen wird.

9. Spender nach Anspruch 5, wobei der Betätigungsmechanismus einen Verankerungsblock (44) aufweist, welcher so beschaffen ist, dass er die Ventilspindel (12) hält und eine Bewegung der Ventilspindel relativ zu dem Adapter (30) verhindert, und wobei der Behälter (25) in Richtung der Ventilspindel vorgespannt ist, derart, dass die Ventilspindel in ihrer geschlossenen oder Grundposition verbleibt, und wobei der Betätigungsmechanismus ferner ein Betätigungselement (35) aufweist, wobei das Betätigungselement derart gestaltet ist, dass bei Einwirkung der Druck- oder Quetschkraft das Betätigungselement den Behälter entgegen der Vorspannung von der Ventilspindel weg bewegt, wodurch es die Ventilspindel in ihre Spendeposition relativ zu dem Behälter zieht.

10. Spender nach einem der Ansprüche 4 bis 9, wobei das Abgabeventil (15) derart gestaltet ist, dass die Ventilspindel (12) zu ihrer Spendeposition hin durch den Dampfdruck vorgespannt wird, der durch die unter Druck stehende Aerosol-Formulierung erzeugt wird.

11. Spender nach einem der Ansprüche 4, 5, 6, 8 und 9, wobei das Abgabeventil (15) eine neutrale Vorspannung aufweist.

12. Spender nach Anspruch 7, wobei das Abgabeventil (15) eine innere Vorspannfeder (39) aufweist, die am inneren Ende der Ventilspindel angeordnet ist und die Ventilspindel in Richtung ihrer Spendeposition vorspannt.

13. Spender nach einem der Ansprüche 4 bis 12, wobei das Abgabeventil (15) ein Ventil zur Abgabe genau dosierter Mengen ist.

14. Spender nach Anspruch 13, wobei das Abgabeventil (15) ferner eine Kammer (6) und einen Auslasskanal (10) aufweist und die Ventilspindel (12) sich in die Kammer hinein erstreckt und relativ zu der Kammer zwischen der geschlossenen oder Grundposition und der Spendeposition beweglich ist, wobei die Ventilspindel eine Konfiguration hat, die eine Außenfläche aufweist, und die Kammer eine innere Konfiguration hat, die eine Innenfläche aufweist, derart, dass ein bewegliches dosiertes Volumen von unter Druck stehender Aerosol-Formulierung dazwischen definiert werden kann, und derart, dass während der Bewegung zwischen der geschlossenen oder Grundposition und der Spendeposition die Ventilspindel nacheinander:
(i) eine freie Strömung von Aerosol-Formulierung in die Kammer hinein und aus ihr hinaus ermöglicht,
(ii) ein abgeschlossenes dosiertes Volumen von unter Druck stehender Aerosol-Formulierung zwischen der Außenfläche der Ventilspindel und der Innenfläche der Kammer definiert, und
(iii) das abgeschlossene dosierte Volumen innerhalb der Kammer bewegt, ohne das Volumen des abgeschlossenen dosierten Volumens zu verringern, bis das dosierte Volumen mit dem Auslasskanal kommuniziert, und dadurch das Spenden des dosierten Volumens von unter Druck stehender Aerosol-Formulierung ermöglicht.

15. Spender nach einem der Ansprüche 4 bis 14, wobei die unter Druck stehende Aerosol-Formulierung ein Medikament umfasst.

16. Spender nach einem der Ansprüche 4 bis 15, wobei die unter Druck stehende Aerosol-Formulierung ein Treibgas umfasst.

17. Spender nach Anspruch 16, wobei das Treibgas ein Hydrofluoralkan ist.

18. Spender nach Anspruch 17, wobei das Treibgas aus HFA 134a, HFA 227 und Mischungen davon gewählt ist.

19. Spender nach einem der Ansprüche 4 bis 18, wobei der Spender die Form eines Inhalators hat.

## Revendications

1. Adaptateur (30) pour une utilisation avec un récipient (25) équipé d'une valve de diffusion (15) qui comporte une tige de valve (12) mobile entre une position intérieure fermée ou d'amorçage et une position de diffusion extérieure, pour diffuser des doses d'une formule d'aérosols sous pression,
l'adaptateur étant adapté pour recevoir le récipient et comportant un mécanisme d'actionnement, **caractérisé en ce que** ledit mécanisme d'actionnement est disposé de telle manière que l'utilisateur fera fonctionner le mécanisme en appliquant une force d'enfoncement ou de serrage et la dose sera diffusée au moment où ladite force d'enfoncement ou de serrage est exercée et dans lequel ledit mécanisme d'actionnement est disposé de telle manière que, au moment où le mécanisme est relâché par l'utilisateur, la tige de valve de la valve de diffusion se déplacera automatiquement vers sa position fermée ou d'amorçage.

2. Adaptateur selon la revendication 1, dans lequel le mécanisme d'actionnement est disposé de telle manière que, au moment où ladite force d'enfoncement ou de serrage est exercée, la tige de valve (12) sera tirée depuis sa position fermée ou d'amorçage jusqu'à sa position de diffusion.

3. Adaptateur selon la revendication 1 ou 2, dans lequel le mécanisme d'actionnement comporte un bloc de montage (34) et un actionneur (35) ; ledit bloc de montage étant adapté pour retenir le récipient (25) et pour empêcher le déplacement du récipient par rapport à l'adaptateur (30) et ledit actionneur étant disposé de façon à toucher la tige de valve (12) et être sollicité vers le récipient, de sorte que la tige de valve reposera dans sa position fermée ou d'amorçage.

4. Diffuseur (100) pour diffuser des doses de formule d'aérosols sous pression à un utilisateur, ledit diffuseur comportant :
un récipient (25) contenant une formule d'aérosols sous pression et équipé d'une valve de diffusion (15) qui comporte une tige de valve (12) mobile entre une position intérieure fermée ou d'amorçage et une position de diffusion extérieure, pour diffuser une dose ;
et
un adaptateur (30) adapté pour recevoir le récipient et comportant un mécanisme d'actionnement, **caractérisé en ce que** ledit mécanisme d'actionnement est disposé de telle manière que l'utilisateur fait fonctionner le mécanisme en appliquant une force d'enfoncement ou de serrage et la dose est diffusée au moment où ladite force d'enfoncement ou de serrage est exercée et dans lequel ledit mécanisme d'actionnement est disposé de telle manière que, au moment où le mécanisme est relâché par l'utilisateur, la tige de valve de la valve de diffusion est déplacée automatiquement vers sa position fermée ou d'amorçage.

5. Diffuseur selon la revendication 4, dans lequel le mécanisme d'actionnement est disposé de telle manière que, au moment où ladite force d'enfoncement ou de serrage est exercée, la tige de valve est tirée depuis sa position fermée ou d'amorçage jusqu'à sa position de diffusion.

6. Diffuseur selon la revendication 4 ou 5, dans lequel le mécanisme d'actionnement comporte un bloc de montage (34) et un actionneur (35) ; ledit bloc de montage étant adapté pour retenir le récipient (25) et pour empêcher le déplacement du récipient par rapport à l'adaptateur (30) et ledit actionneur touchant la tige de valve (12) et étant sollicité vers le récipient, de sorte que la tige de valve repose dans sa position fermée ou d'amorçage.

7. Diffuseur selon la revendication 6 telle qu'elle dépend de la revendication 4, dans lequel ledit actionneur (35) est disposé de telle manière que, au moment où ladite force d'enfoncement ou de serrage est exercée, l'actionneur s'éloigne du récipient (25) contre la sollicitation, permettant à la tige de valve (12) de se déplacer vers sa position de diffusion.

8. Diffuseur selon la revendication 6 telle qu'elle dépend de la revendication 5, dans lequel ledit actionneur (35) est accouplé à la tige de valve (12) et dans lequel ledit actionneur est disposé de telle manière que, au moment où ladite force d'enfoncement ou de serrage est exercée, l'actionneur s'éloigne du récipient (25) contre la sollicitation et la valve est tirée vers sa position de diffusion.

9. Diffuseur selon la revendication 5, dans lequel le mécanisme d'actionnement comporte un bloc d'ancrage (44), qui est adapté pour retenir la tige de valve (12) et pour empêcher le déplacement de la tige de valve par rapport à l'adaptateur (30), et dans lequel le récipient (25) est sollicité vers la tige de valve, de sorte que la tige de valve repose dans sa position fermée ou d'amorçage, et dans lequel ledit mécanisme d'actionnement comporte en outre un actionneur (35), ledit actionneur étant disposé de telle manière que, au moment où ladite force d'enfoncement ou de serrage est exercée, l'actionneur déplace le récipient contre la sollicitation en l'éloignant de la tige de valve, tirant ainsi la tige de valve vers sa position de diffusion par rapport au récipient.

10. Diffuseur selon l'une quelconque des revendications 4 à 9, dans lequel la valve de diffusion (15) est disposée de telle manière que la tige de valve (12) est sollicitée vers sa position de diffusion par la pression de vapeur générée par la formule d'aérosols sous pression.

11. Diffuseur selon l'une quelconque des revendications 4, 5, 6, 8 et 9, dans lequel la tige de diffusion (15) est d'une sollicitation neutre.

12. Diffuseur selon la revendication 7, dans lequel la valve de diffusion (15) comporte une sollicitation à ressort interne (39) positionnée à l'extrémité intérieure de la tige de valve sollicitant la tige de valve vers sa position de diffusion.

13. Diffuseur selon l'une quelconque des revendications 4 à 12, dans lequel la valve de diffusion (15) est une valve de diffusion de doses mesurées.

14. Diffuseur selon la revendication 13, dans lequel la valve de diffusion (15) comporte en outre une chambre (6) et un passage menant à un orifice de sortie (10), et la tige de valve (12) se prolonge jusque dans la chambre et est mobile par rapport à la chambre entre la position fermée ou d'amorçage et la position de diffusion, la tige de valve ayant une configuration incorporant une surface externe et la chambre ayant une configuration interne incorporant une surface interne, de sorte qu'un volume mesuré mobile d'une formule d'aérosols sous pression est capable d'être défini entre les deux et de sorte que, durant le déplacement entre la position fermée ou d'amorçage et la position de diffusion, la tige de valve séquentiellement :
(i) laisse couler librement la formule d'aérosols dans la chambre et hors de la chambre,
(ii) définit un volume mesuré fermé pour la formule d'aérosols sous pression entre la surface externe de la tige de valve et la surface interne de la chambre, et
(iii) se déplace avec le volume mesuré fermé à l'intérieur de la chambre sans diminuer le volume du volume mesuré fermé jusqu'à ce que le volume mesuré communique avec le passage menant à l'orifice de sortie, permettant ainsi la diffusion du volume mesuré de formule d'aérosols sous pression.

15. Diffuseur selon l'une quelconque des revendications 4 à 14, dans lequel la formule d'aérosols sous pression comporte un médicament.

16. Diffuseur selon l'une quelconque des revendications 4 à 15, dans lequel la formule d'aérosols sous pression comporte un propulseur.

17. Diffuseur selon la revendication 16, dans lequel le propulseur est un hydrofluoroalcane.

18. Diffuseur selon la revendication 17, dans lequel le propulseur est sélectionné dans le groupe comprenant du HFA 134a, du HFA 227 et des mélanges associés.

19. Diffuseur selon l'une quelconque des revendications 4 à 18, dans lequel le diffuseur se présente sous la forme d'un inhalateur.
